(19)

Europäisches
Patentamt
European
Patent Office
Office européen
des brevets

(11) **EP 2 017 349 A1**

(12) **EUROPEAN PATENT APPLICATION**

(43) Date of publication:
**21.01.2009 Bulletin 2009/04**

(51) Int Cl.:
*C12P 19/02* (2006.01)  *C12S 3/04* (2006.01)
*C13K 1/02* (2006.01)  *C13K 1/06* (2006.01)
*C07H 1/08* (2006.01)  *C12P 19/12* (2006.01)
*C12P 19/14* (2006.01)  *C12P 19/16* (2006.01)
*C12P 19/18* (2006.01)  *C12P 19/20* (2006.01)
*C12P 19/22* (2006.01)  *C12P 19/24* (2006.01)

(21) Application number: **07011507.6**

(22) Date of filing: **12.06.2007**

(84) Designated Contracting States:
**AT BE BG CH CY CZ DE DK EE ES FI FR GB GR
HU IE IS IT LI LT LU LV MC MT NL PL PT RO SE
SI SK TR**
Designated Extension States:
**AL BA HR MK RS**

(71) Applicant: **SÜD-CHEMIE AG
80333 München (DE)**

(72) Inventors:
• **Koltermann, Andre
82057 Icking (DE)**

• **Kettling, Ulrich
80797 München (DE)**
• **Brück, Thomas
82067 Ebenhausen (DE)**
• **Rarbach, Markus
81369 München (DE)**

(74) Representative: **Westendorp, Michael Oliver et al
Splanemann Reitzner
Baronetzky Westendorp
Rumfordstraße 7
80469 München (DE)**

(54) **Generation of chemical building blocks from plant biomass by selective depolymerization**

(57)  The invention concerns a method for the enzymatic treatment of raw polymeric feedstock comprising the following steps: (a) preferably separation of soluble components from the raw polymeric feedstock, (b) treating the raw polymeric feedstock with an enzyme system in order to liberate defined soluble monomeric or oligomeric building blocks from the insoluble raw polymeric feedstock; and (c) separating the defined monomeric or oligomeric building blocks produced in step b) from the remainder of the raw polymeric feedstock. Preferably, the enzyme system used in step b) contains not more than 50%, preferably not more than 20%, more preferably not more than 10%, more preferably not more than 5%, more preferably not more than 2%, more preferably not more than 1% of other enzyme activities apart from the enzyme activity resulting in liberation of said defined monomeric or oligomeric building blocks from the raw polymeric feedstock according to step b). Further aspects of the invention concern the use of "less pure" and thus less costly enzyme systems in subsequent enzymatic treatment steps and methods for determining the optimum sequence of enzymatic treatment steps by analysis of the raw polymeric feedstock used.

**FIG. 1**

EP 2 017 349 A1

**Description**

**Background of the invention**

The generation of biobased chemical building blocks from renewable resources has recently attracted increasing attention due to the global limitation of fossil petrochemical resources. The preferred feedstocks for the generation of such biobased chemical products are derived from renewable plant biomass (Kamm *et al.,* 2006).

The current manufacturing processes for biobased products predominantly utilize substrates from the food and feed market such as oils, sugars, and starches. Most first generation feedstocks are of well-defined chemical composition and low structural complexity. Additionally, these substrates can be obtained in relatively high purity with only minor amounts of accompanying contaminants. While their use is both technologically and economically appealing, their continuing large-scale supply is not secure because the use of first generation feedstocks in biobased chemical processes is in fierce competition with their ever increasing global demand in the food industry.

Alternative substrates of the aforementioned first generation feedstocks are derived from low cost forestry by-products and agricultural wastes constituting plant material that has no application as a food source. Examples of these designated second generation feedstocks are residual plant material from farming activities such as corn stover and wheat straw as well as various wood related wastes. This Lignocellulosic Biomass (LCB) is distinguished from first generation biological feedstocks by its complex chemical and structural composition. The primary components of LCB are highly polymeric materials such as cellulose (approx. 35-50% w/w), hemicellulose (approx. 20-35% w/w), and lignins (approx. 10-25% w/w). Proteins, lipids and other compounds constitute minor fractions in most LCB raw materials (Saha, 2005) but can be present in larger quantities in special agricultural wastes such as residues from oil production. Since LCB is composed of multiple, chemically diverse components, its downstream processing is technically difficult, which in turn limits the economic feasibility of current LCB-based bioprocesses.

**Technical Problem**

In order to produce valuable chemical substances and building blocks via economically viable bioprocesses based on LCB feedstocks, it is important to both (i) recover and refine the majority of its diverse chemical constituents and (ii) to produce them in sufficient purity. In contrast to the diverse and highly polymeric entities that make up LCB, its favored processing products are of low molecular weight. Principally, economically attractive products can be generated from all components of LCB: Glucose generated from cellulose is a versatile starting material for the generation of high-value chemical intermediates such as sorbitol. Pentose sugars such as xylose and arabinose deduced from hemicellulose fractions of LCB are starting materials for high-value low-nutritional and non-calorigenic sweeteners such as xylitol and arabinitol. Proteins from LCB can be hydrolyzed to yield enantiopure L-amino acids. Lignins can serve as a source of phenolic compounds substituting for aromatics produced by petrochemical processes. The current technological limitation for the use of second generation feedstocks is mainly associated with their complex chemical composition.

Most current processes that use LCBs concentrate on the cellulose part of the substrate. When other components are used they are typically hydrolyzed by unselective process steps such as pretreatment with sulphuric acid hydrolyzing all hemicelluloses in a single process step. The products of these unselective process steps are generally of low and in some cases negative commercial value.

The presently operating logen process for the fermentative production of bioethanol uses dilute acid steam pretreatment at 200-250°C to mobilize the hemicellulose fraction of LCBs, which contains a mixture of different hexose and pentose sugars. In a separate enzymatic step the insoluble cellulose fraction is hydrolyzed to hexose (glucose) sugars. After liquefaction of the hemicellulose and cellulose fractions, insoluble lignin solids are physically separated from the marsh and burned to generate energy for downstream processing of the remaining LCB fractions. The combined cellulose and hemicellulose fractions are fermented together in a single step to produce bioethanol. The resulting ethanol is subsequently recovered from the fermentation broth by distillation. Since the majority of commercially available organisms (i.e. baker's yeast, *Saccharomyces cerevisiae)* used for the fermentation process are unable to utilize pentose sugars, these components of LCBs, albeit of significant commercial value when present in pure form, are discarded in the process together with remaining waste residue (Lawford and Rousseau, 2003).

Recently, attempts have been made to make the pentose fraction of LCBs available for the fermentative conversion to bioethanol. In this revised process design, the liquefied hemicellulose fraction is separated from the remaining components of LCB after the pretreatment step. While all remaining LCB fractions are processed as previously described, specially engineered microorganisms (i.e. engineered strains of *Zymomonas mobilis)* with the ability to utilize pentose (Lawford and Rousseau, 2003; Lynd *et al.,* 2005) as well as hexose sugars are employed in a separate fermentation step to effectively convert the liquefied and conditioned hemicellulose to bioethanol. The resulting fermentation marsh is subsequently fed into the conventional process stream to recover the bioethanol. While bioethanol production from complex pentose mixtures seems to be commercially valuable, the selective processing of hexose, pentose, and lignin to high-value products and chemical building blocks is an attractive alternative route for the utilization of LCB components.

One inherent problem of all currently used pretreatment methods is the simultaneous and non-selective hydrolysis and release of various chemical building blocks that make up LCB components (Saha, 2005). At present, commercially applied and economically viable pretreatment methods employ harsh chemical or physical treatments, which may include a combination of acid or base treatments at elevated temperatures (Ramos *et al.,* 2005). The resulting LCB hydrolysates contain a variety of unwanted by-products derived from chemical modification of LCB building blocks. The presence of these contaminants often precludes downstream enzymatic or catalytic processing or whole-cell fermentation of the products (Saha, 2005) and therefore seriously lowers the commercial value of product streams generated from LCBs by such methods.

Thus, the technical problem underlying the present invention is to provide a method for the production of chemical building blocks from renewable plant biomass.

Especially, the technical problem is to provide a method for the production of valuable chemical building blocks from LCB, which avoids the disadvantages and drawbacks of the prior art.

## Summary of the invention

According to a first aspect, the present invention provides a treatment method for the enzymatic treatment of raw polymeric feedstock comprising the following steps: (a) treating the raw polymeric feedstock with an enzyme system in order to liberate defined monomeric or oligomeric building blocks from the raw polymeric feedstock and (b) separating the defined monomeric or oligomeric building blocks produced in step a) from the remainder of the raw polymeric feedstock.

According to a preferred aspect, steps a) and b) are performed in a solvent (liquid medium). Preferably, the (raw) polymeric feedstock is treated in the presence of a solvent in which it is insoluble, i.e. in which it is not present in dissolved form. Thus the (raw) polymeric feedstock preferably is an insoluble raw polymeric feedstock. The solvent is preferably an aqueous solvent. Further preferred, the enzyme step a) liberates soluble monomeric or oligomeric building blocks from the raw polymeric feedstock, i.e. monomeric or oligomeric building blocks, which are soluble in the solvent used and can thus be dissolved therein. According to a further preferred aspect, the separation of the soluble (dissolved) defined monomeric or oligomeric building blocks produced in step a) from the remainder of the insoluble (not dissolved) raw or processed polymeric feedstock (step b) is achieved by solid-liquid separation. Any conventional method for solid-liquid separation may be used, including filtration or centrifugation methods.

According to another preferred aspect, the invention comprises either a single consolidated process consisting of step a) and step b) or a series of sequential process steps, wherein step a) and step b) are repeated at least once. In each process step, soluble monomeric or oligomeric products are produced from insoluble raw or processed polymeric feedstock by successive addition of a specific enzyme system followed by the separation of the soluble monomeric or oligomeric products from the insoluble remainder of the polymeric feedstock. Any conventional method for solid-liquid separation may be used, including filtration or centrifugation methods.

According to a preferred aspect, the defined monomeric or oligomeric building block(s) liberated from the raw or processed polymeric feedstock in every treatment step a) is one specific "product" selected from the left column of Table 1 below. In other words, only one specific monomeric or polymeric building block is liberated from the raw or processed polymeric feedstock in every treatment step a) using an enzyme system or a combination of enzyme systems having the same product. Examples of such combinations of enzymes are listed in Table 1.

According to a particularly advantageous aspect of the invention, the treatment method of the invention comprises at least one treatment step ("pretreatment step") prior to step a) for separating (removing) soluble components from the raw or processed (insoluble) polymeric feedstock. Thus, this step is performed prior to the enzymatic treatment of the raw or processed polymeric feedstock. It has been found that the efficiency of the subsequent enzymatic treatment step(s) can be surprisingly increased by such pretreatment step(s) for removing soluble components from the raw or processed polymeric feedstock. The preferred conditions of such pretreatment step(s) are further discussed below.

According to a preferred embodiment, the same or a similar solvent (liquid medium) as used for the following enzymatic treatment step a) is used in the pretreatment step(s). Individual pretreatment step(s) for the removal of solubles are preferably performed at the same and more preferably at higher temperatures as the following enzymatic treatment step a) to increase the extraction efficiency. Even more preferably, soluble extraction by the pretreatment step(s) will be performed at higher temperatures and pressures (pressure cooker principle) but at a shortened treatment time compared to the following enzymatic treatment step. Again, the raw or processed polymeric feedstock is preferably insoluble in the solvent used and the components to be removed are soluble therein. Separation of the soluble components from the insoluble raw or processed polymeric feedstock is preferably performed by conventional solid-liquid separation methods.

According to another embodiment of the invention, the individual pretreatment step(s) for removing soluble components from the (raw or processed) polymeric feedstock prior to step a) can be repeated in multiple stepwise cycles of at least two pretreatment steps. In a preferred embodiment, said cycles are carried out with varying solvent compositions, temperature and time profiles to increase the efficacy of soluble extraction.

The pretreatment step(s) can comprise one or more physico-chemical pretreatment step and/or one or more washing step as defined herein.

In the present invention, it has been found according to one preferred aspect that the separation of the liberated (soluble) monomeric or oligomeric building block(s) from the (insoluble) raw or processed polymeric feedstock after a defined treatment with an enzyme system provides significant advantages regarding the generation of chemically pure value-added base chemicals and chemical building blocks from complex natural substrates such as LCB by employing selective catalytic process steps. These selective catalytic process steps liberate defined chemical monomeric or oligomeric components from the complex polymeric feedstock with low amounts of other contaminants in the product generated by the process step. A preferred technical solution to provide such specificity and selectivity in the hydrolysis of LCB is the use of selective enzymatic steps. The base chemicals can be used as substituents for starting material in traditional petrochemical processes as well as starting materials for novel chemical and biochemical synthesis routes and are therefore of high commercial value.

Further preferred aspects and embodiments are described in detail below.

**Definitions**

The term "raw polymeric feedstocks" means complex mixtures of different insoluble polymeric substrates such as carbohydrates, polymeric lipids, polypeptides, polynucleotides, and polymeric phenylpropanoids in varying mass ratios that are usually derived from plant material. In addition to these insoluble polymeric substrates raw polymeric feedstocks usually contain soluble monomeric or oligomeric components. Raw polymeric feedstocks include but are not limited to waste products from the forestry, agricultural and food processing industry as well as municipal waste. When the main polymers are cellulose and lignin such raw polymeric feedstocks are termed "raw lignocellulosic feedstocks" or "lignocellulosic biomass" or "LCB". Raw lignocellulosic feedstocks from agricultural activities comprise but are not limited to wheat straw, corn stover, rumen animal manure, sugarcane bargass, sugar beet pulp, and herbaceous material like switch grass, Sericea Lespedeza Serala and Sorghum sudan grass. Forestry derived waste feedstocks comprise but are not limited to wood bark, wood chips, and waste timber. Lignocellulosic feedstocks derived from the food industry encompass but are not limited to fruit pulp, agave whole residue, coffee residue, and oil mill waste such as rapeseed press cake and mill effluents. Raw feedstocks derived from the pulp and paper industry include but are not limited to paper sludge and paper mill effluents. Raw feedstocks derived from municipal waste encompass but are not limited to waste paper, vegetable residue, and fruit residue.

The term "processed polymeric feedstock" as used herein shall mean the (remainder of the) raw polymeric feedstock after at least one enzymatic treatment step (step a).

The term "washing step" shall mean any pretreatment step of the raw or processed polymeric feedstock using at least one solvent in order to extract soluble components from the insoluble polymeric feedstock without modifying or changing the structure of the polymeric feedstock itself.

The term "physico-chemical treatment step" shall mean any pretreatment step of the raw or processed polymeric feedstock in order to extract soluble components from the insoluble polymeric feedstock including modifying or changing the structure of the polymeric feedstock itself.

The term "polymeric substrate" means substances composed of either a specific monomeric constituent or a limited variety of defined monomeric constituents covalently linked together in a linear or partially branched molecular structure. The insoluble fraction of raw lignocellulosic feedstocks contains significant amounts of such polymeric substrates such as cellulose, xylan, mannan, and galactan. Additionally, it also contains polymeric substrates such as lignin, arabinoxylan, glucoronoxylan, glucomannan, and xyloglucan.

The term "enzyme systems" means proteinaceous entities that are able to catalytically convert polymeric or oligomeric substrates into smaller oligomeric or monomeric constituents (building blocks). In addition to the use of a single enzyme to produce monomeric or oligomeric products from a polymeric substrate, the term enzyme system also comprises mixtures comprising more than one enzyme that produce a defined monomeric or oligomeric product by synergistic or parallel action from a polymeric feedstock. Thus, the terms "enzyme system" and "enzyme mixtures" are used interchangeably herein and both may comprise one or more enzyme or enzyme activity, respectively.

The term "monomeric or oligomeric building blocks" means monomeric or oligomeric products, which are liberated from the raw polymeric feedstock using an enzyme system. "Oligomeric" shall include compounds with two or more monomeric units.

The term "enzymatic activity" of an enzyme refers to the enzyme's catalytic activity under appropriate conditions under which the enzyme serves as a protein catalyst, which converts specific polymeric or artificial substrates to specific oligomeric or monomeric products.

The term "contaminating enzymatic activity" describes enzymatic activities of an employed enzyme system that lead to oligomeric or monomeric products other than the desired oligomeric or monomeric product(s), which are produced according to the intended (main or first) enzymatic activity of the enzyme system.

The term "artificial substrate" means a substance of commonly low molecular weight that after contacting the artificial substrate with an enzyme system gives a measurable change in the physico-chemical property of the artificial substrate that correlates to the activity of a selected enzyme system. Said physico-chemical properties and artificial substrates to give such changes in physico-chemical properties after contacting them with an enzyme system are known to those skilled in the art and comprise but are not limited to changes in spectrophotometrically measurable absorption or fluorescence emission properties, changes in chromatographic mobility to be determined by liquid or gas chromatograph and changes in molecular mass to be determined by mass spectroscopy.

Suitable artificial substrates for enzymes, enzyme systems, and measurable reaction product are listed in Table 2 below.

The term "liberate" means the conversion of an insoluble polymeric substrate to a soluble monomeric or oligomeric product by a physical, chemical, or catalytic process such as hydrolysis, oxidative or reductive depolymerization.

**Detailed description of the invention**

*General*

The invention comprises according to a first aspect a single consolidated process or a series of sequential process steps for the generation of base chemicals or chemical building blocks from a raw polymeric feedstock. In each process step soluble monomeric or oligomeric products (building blocks) are produced from insoluble raw or processed polymeric feedstocks by contacting the raw or processed polymeric feedstock with a specific enzyme system, which is preferably essentially free of enzymatic activities producing other than the intended reaction product (monomeric or polymeric building block), followed by the separation of the soluble monomeric or oligomeric building block(s) from the insoluble raw or processed polymeric feedstock.

According to a preferred embodiment, the method comprises the separation of soluble components from (insoluble) polymeric feedstocks such as LCB prior to or in combination with the single consolidated process or prior to and in combination with one or more step(s) of a series of sequential (enzymatic) process steps as defined in the preceding paragraph.

According to another preferred embodiment of the invention, the initial separation of the soluble components from the (insoluble) raw polymeric feedstock prior to the enzymatic treatment steps comprises at least one physico-chemical pretreatment step followed by solid-liquid separation. Said physico-chemical treatment step(s) may include but are not limited to incubating the raw polymeric feedstock with a solvent under increased temperature and/or increased pressure and/or contacting the raw polymeric feedstocks with chemicals. Such chemicals include but are not limited to dilute or concentrated acids or bases. Preferably, the physico-chemical pretreatment is performed at a pH of 1-13 and more preferably a pH of either 2-5 or 8-11. According to a further preferred embodiment, the physico-chemical pretreatment is combined with one or more washing steps, preferably in order to further reduce soluble components in the raw polymeric feedstock.

According to a preferred embodiment, the pretreatment step(s) as defined herein are performed at a temperature in the range from 20 to 210°C, preferably at 50-175°C. The pressure range for each individual pretreatment step may range from 1 to 300 bar and preferentially will be in the range from 1 to 100 bar. The preferred duration of the pretreatment step(s) is dependent on the composition of the raw or processed polymeric feedstock and may range from few seconds to 1 week. Most preferentially, the treatment time for each individual pretreatment step may not exceed 1 h.

Said washing step(s) prior to the enzymatic treatment step(s) comprise at least one washing step with at least one solvent, preferably at least one aqueous solvent. Most preferred are, without limitation water or aqueous buffers. Preferably, the washing step is followed by a solid-liquid separation. According to one embodiment, the washing step is performed in the absence of an enzyme or catalyst.

In a preferred embodiment of the invention, the liquid medium used for the individual pretreatment step(s), in particular washing step(s), contains varying concentrations of inorganic salts and/or other chemical components that may affect ionic strength, pH and/or hydrophobicity of the medium to increase extractability of soluble material preferably before and after each enzymatic treatment step. Preferably, the liquid medium used for the individual pretreatment steps is characterized by a pH of 1-13. Preferably the liquid medium used for the individual pretreatment steps contains inorganic acid, bases and salts such as hydrogen chloride, sulphuric acid, ammonia, sodium chloride, sodium hydroxide, ammonium sulphate, sodium phosphate, sodium acetate, sodium citrate, sodium tartrate, sodium sulphate, and/or organic buffer components, e.g. glycine, glycerol, and/or Triton-X100 for hydrophobicity modification. Preferably, the ionic strength of the liquid medium used for the individual pretreatment steps is in the range of 0.1-10M equivalents of sodium sulphate (Ionic strength I~ 1.7-170). In another preferred embodiment, the liquid medium is free of any organic solvent and/or compound that is not water-miscible.

In yet another preferred embodiment of the invention, individual washing step(s) are applied repeatedly to the raw or processed polymeric feedstock with varying solvent compositions using varying time, temperature and pressure profiles to maximize the extraction of a particular soluble component.

In a preferred embodiment of the invention, at least one physico-chemical pretreatment step prior to the enzymatic treatment of the raw polymeric feedstock is designed to (primarily) remove lignin, resin and/or proteinaceous components from the raw polymeric feedstock. Preferably, a liquid medium with an ionic strength (I) of more than 1 is used and, in another preferred embodiment, all subsequent pretreatment step(s), in particular washing step(s), are performed using a liquid medium with lower ionic strength than in the initial physico-chemical pretreatment step.

Methods for separation of soluble and insoluble components are known to the person skilled in the art and comprise process steps such as sedimentation, decantation, filtration, micro-filtration, ultra-filtration, centrifugation, evaporation of volatile products, and extraction with organic or inorganic solvents. According to a preferred embodiment, the pretreatment step(s) comprises a treatment with aqueous solvents, organic solvents, or any combination or mixtures of these preferably with ethanol or glycerol.

According to a preferred embodiment of the invention, the enzymatic treatment is performed in an aqueous medium, the defined monomeric or oligomeric building blocks liberated from the raw or processed polymeric feedstock are soluble in the aqueous medium, and the separation according to step b) above is performed by solid-liquid separation of the soluble building blocks in the aqueous medium from the insoluble raw or processed polymeric feedstock.

Relevant examples of polymeric substrates, which constitute major components of polymeric feedstocks, their monomeric or oligomeric degradation products (building blocks), and enzyme systems useful for generating essentially pure products from each polymeric substrate contained in a raw or processed polymeric feedstock are listed in Table 1 below.

In the processing steps of the invention, chemical building blocks of polymeric substrates like hemicellulose, cellulose, lignins, glucans, proteins, and lipids are released by contacting the polymeric feedstock with specific enzyme systems. The enzymatic decomposition of individual feedstock constituents into essentially pure soluble products is based on the application of substrate-specific enzyme preparations, which are, according to a preferred embodiment, essentially free of activities that release other constituents than the intended products in the respective process step.

*Determination of composition of raw polymeric feedstock*

The molecular composition of the raw feedstocks to be employed in the herein described processes can be determined by methods known to those skilled in the art. For example, the composition of lignocellulosic material may be determined using a combination of pyrolysis, gas chromatography, and mass spectrometry. A library of methods describing possible analytical methodologies for the determination of LCB components is listed under:
http://www1.eere.energy.gov/biomass/analytical procedures html#samples.

According to one embodiment, standard procedures comprising several physical and enzymatic reaction steps may be employed to empirically quantify the constituents of the polymeric feedstock on the basis of the obtained reaction products.

For common feedstocks a database listing the mass ratios for the most common feedstock classes can be found under:
http://www1.eere.energy.gov/biomass/feedstock databases.html.

In an illustration to analyze the feedstock components, the raw polymeric feedstock has first to be partially hydrolyzed before further analysis can be conducted.

Prior to feedstock hydrolysis, the raw feedstock sample (1 g) should be placed into a crucible and dried at 50°C until a constant weight is obtained. The dry weight is recorded to 3 decimal places to obtain the oven dry weight (ODW) of the sample.

For the hydrolysis procedure, 1g of finely milled feedstock ($2\mu m$) is placed in a pressure tube and 3ml of 72% v/v sulphuric acid is added. The pressure tube is set in a water bath at 30°C and incubated for 1 h. Using a stirring rod the sample has to be stirred every 5 to 10 min without removing the sample from the bath. Stirring is essential to ensure even acid to particle distribution. The acid is diluted to 4% by adding 84ml double distilled (d.d.) water using a burette and the sample is mixed by inverting the tubes several times to eliminate phase separation.

In order to determine the acid-insoluble lignin fraction of the feedstock, the autoclaved hydrolysis solution is vacuum filtered through a previously weighed filtering crucible.

The filtrate is captured in a filtering flask and an aliquot of 50ml is transferred into a sample storage bottle. This sample will be used in the following procedure to determine the lignin and carbohydrate content. The acid-soluble lignin determination must be performed within six hours of hydrolysis.

For the determination of acid-insoluble lignin, d.d. water is added to quantitatively transfer all remaining insolubles out of the pressure tube into the filtering crucible. The insolubles have to be rinsed with a minimum of 50ml d.d.

water and subsequently the crucible and acid-insoluble residues have to be dried at 105°C for 4h or until a constant weight is obtained. After incubation the samples are removed from the oven and cooled in a dessicator. The weight "w2" of the crucible and dry residue have to be recorded to the nearest 0.1 mg before the crucible and residue are placed in a muffle furnace at 575°C for 24h.

The crucible is carefully removed from the furnace and transferred directly into a dessicator and cooled for a specific amount of time that is equal to the initial cooling time of the crucible. The crucible and ash are weighed (weight "w3") and placed back into the furnace until a constant weight is obtained. The weight "w2" corrected for the remaining ash ("w3") is equal to the weight of acid-insoluble lignin contained in the raw feedstock.

In contrast to the complex measurements required to obtain the amount of acid-insoluble lignin, the amount of acid-soluble lignin can be easily determined spectrophotometrically. First a background measurement is run with aqueous 4% v/v sulphuric acid on a spectrophotometer of choice. Using the initial hydrolysis liquor, the absorbance at 320nm and at the maximal absorbance of the filtrate hydrolysate, which is usually around 198nm, is measured. The sample has to be diluted as necessary to bring the absorbance range to 0.7-1.0 A units. The absorbance of the sample to 3 decimal places is used to calculate the amount of acid-soluble lignin (ASL) present in the sample according to the calculation below:

$$\% \text{ ASL} = (UV_{abs} * Volume_{filtrate} * \text{dilution factor}) / (e * ODW_{sample}) * 100$$

ODW = oven dry weight of raw feedstock sample
$UV_{abs}$ = average UV-vis absorbance for the sample at 320nm
Volume filtrate = Volume of the hydrolysis filtrate
E = Extinction coefficient of biomass hydrolysate liquor at maximal absorbance of sample (numerical values of the extinction coefficients for a large number of raw polymeric feedstocks can be found in
http://www1.eere.energy.gov/biomass/analytical_procedures.html#samples)

The sample hydrolysate liquor may also be used to determine the structural carbohydrates contained within the hemicellulose fraction of the feedstock using an HPLC-based procedure.

This determination first requires a calibration mixture for each D-cellobiose, glucose, xylose, galactose, arabinose, and mannose. The concentrations prepared for each sugar standard should range from 0.1-4mg/ml. For each set of calibration standards, an independent calibration verification standard (CVS) should be prepared that falls in the middle of the validated range of the calibration curve (i.e. 2.5mg/ml). The CVS should be analyzed by HPLC after each calibration set and at regular intervals throughout the analysis sequence, bracketing groups of samples. The CVS is used to verify the quality and stability of the calibration curves of each run.

20ml of the hydrolysis liquor obtained in the initial steps after sample hydrolysis are transferred into a 50ml Erlenmeyer flask. Calcium carbonate is added to neutralize the sample to a pH of 5-6 and after settling of the solution the supernatant can be decanted. After settling the solution will have approximately neutral pH.

The sugar calibration standards CVS and samples are now ready for HPLC analysis using a Shodex® sugar SP0810 (Phenomenex) or an Aminex® HPX-87P (BioRad) column equipped with the appropriate guard column.

The sample injection volume should be between 10 and 50μl dependent on concentration and detector limits. The samples are eluted with d.d. water at a flow rate of 0.6ml/min and a column temperature of 80°C. Sample elution can be monitored best using refractive index detection.

Chromatograms should be integrated prior to analysis and individual sugar contents should be determined with reference to the appropriate standard curves for each saccharide component.

*Enzyme systems*

In the embodiments described herein, the specificities of the applied enzyme mixtures are custom-tailored to obtain pure monomeric product streams (defined monomeric or polymeric building block(s)) derived from different polymeric substrate classes.

In a preferred embodiment of this invention, enzyme systems, applied to a particular raw or processed polymeric feedstock, may contain not more than 50% other enzymatic activities that may result in products other than the preferred product from a designated polymeric feedstock.

In a more preferred embodiment of this invention, enzyme system mixtures, applied to a raw or processed polymeric feedstock, may contain not more than (or less than) 20%, preferably not more than (or less than) 10%, more preferably not more than (or less than) 5%, more preferably not more than (or less than) 2%, most preferably not more than (or less than) 1 % other enzymatic activities that may result in products other than the preferred product from a designated polymeric feedstock.

The percentage of other enzymatic activities may be routinely determined using standard methods for determination of the respective enzyme activity. Thus, the "main" enzyme activity present in the enzyme system leading to liberation of the desired defined monomeric or polymeric building block from the raw or processed polymeric feedstock should amount to at least 50%, preferably at least 80%, more preferably at least 90%, more preferably at least 95%, more preferably at least 98%, more preferably at least 99% of all enzyme activities present in the enzyme system.

The enzyme activities may be determined according to standard methods as known to the skilled person and as described herein.

According to one preferred embodiment, the above percentages may be simply determined by treating the raw polymeric feedstock with the enzyme system according to step a) above and analyzing the liberated soluble monomeric or oligomeric building blocks after solid-liquid separation from the insoluble raw polymeric feedstock according to step b). Thus, if the liberated monomeric or oligomeric building blocks comprise more than 50 mol-% of the specific desired building block (based on the total solids content), the other enzyme activities present in the enzyme system are considered to be less than 50%. Similarly, if the liberated monomeric or oligomeric building blocks comprise more than 80, 90, 95, 98, or 99 mol-% of the specific desired building block, the other enzyme activities present in the enzyme system are considered to be less than 20, 10, 5, 2, or 1 mol-%, respectively. Correspondingly, the "main" enzyme activity present in the enzyme system and leading to liberation of the desired defined monomeric or oligomeric building block from the raw polymeric feedstock is in this case considered to amount to at least 50%, preferably at least 80%, more preferably at least 90%, more preferably at least 95%, more preferably at least 98%, more preferably at least 99%, of all enzyme activities present in the enzyme system. According to a further embodiment, the "mol-%" is replaced by "wt.-%" in the aforementioned determination of the percentage of enzyme activity (activities).

According to one embodiment, enzyme activities can be determined by measuring the rate of conversion of a chosen polymeric substrate as defined above. In an alternative embodiment the enzyme activities present in the enzyme system can be determined by using artificial substrates. Depending on the ease and reliability of the detection methods applied one can either measure the conversion of a substrate itself or alternatively the formation of a specific product resulting from the enzymatically catalyzed reaction. In special cases, catalytic intermediates of the enzyme itself (i.e. oxidoreductases) can be detected spectrophotometrically.

The testing of formulated enzyme mixtures for enzymatic activities is known to those skilled in the art. A library of suitable tests for specific enzyme activities are listed under:

http://www.sigmaaldrich.com/Area of Interest/Biochemicals/Enzyme Explorer/Key Resources/Assay Library/Assays by Enzyme Name.html

Examples of specific tests for determining the enzyme activities of particular enzyme classes are listed below.

In a particular case, both endo- and exo-cellulose activity can be measured in 0.5ml of a 50mM MES (pH 6) reaction buffer containing 10mM $CaCl_2$, 4mM p-nitrophenyl-beta-D-cellobioside and 200$\mu$l of a dilute enzyme solution. The reaction should then be incubated at 50°C for 30min. Glycine buffer (100mM, pH 4) is added to stop the reaction. The enzymatic activity could then be determined by measuring the amount of liberated p-nitrophenol spectrophotometrically at 430nm. The absorbance values of p-nitrophenol are translated to micromoles of nitrophenol using a standard graph relating micromoles of nitrophenol to absorbance. One unit of cellulase activity is the amount of the enzyme required to release 1 $\mu$mol of p-nitrophenol/min under the conditions of the assay (Wood, T.M. and Bhat, K., 1988).

In another particular case, arabinofuranosidase activity can be determined in 1ml of 50mM sodium phosphate (pH 7) containing 4-100$\mu$M 4-nitrophenyl-alpha-L-arabinofuranoside (pNP-Araf) and dilute enzyme solution (4nM-8$\mu$M). The reaction can be incubated at 37°C and the amount of liberated 4-nitrophenol measured at 400nm. Enzyme activity can be calculated using the extinction coefficient 10500$M^{-1}cm^{-1}$ of 4-nitrophenol at 400nm (Taylor et al., 2006).

In another particular case, xylosidase activity can be determined using an o-nitrophenol substituted-beta-D-xylopyranoside (ONP-beta-D-xylopyranoside) as a substrate (Chen et al., 1986). The substrate stock solution (10mM) is prepared in 100mM citrate buffer (pH 5). The dilute enzyme solution to be tested is prepared in d.d. water. The reaction containing equal molar amounts of substrate and enzyme solution in a 200mM borate buffer at 25°C and pH 9.8. To determine the enzyme activity, the liberation of the o-nitrophenol is recorded spectrophotometrically at a wavelength of 410nm. The enzyme activity in units/mg of enzyme is proportional to the liberated amount of o-nitrophenol and can be calculated as described for the activity of cellulase.

In another particular case, laccase activity is determined using the guiacol oxidation assay. A stock solution of 10mM guiacol is freshly prepared in 50mM citrate buffer (pH 4.3, 40°C). The reaction is carried out in 2ml of 50mM citrate buffer using 10$\mu$l of dilute enzyme stock (1-3nM) and varying amounts of substrate (5-20$\mu$l). The rate of guiacol oxidation is then measured spectrophotometrically at 465nm. The rate of guiacol oxidation can be determined using the extinction coefficient of 5200 $M^{-1}cm^{-1}$ for guiacol oxidation products (Smirnov et al. 2001).

In another particular case, manganese peroxidase (MnP) activity can be measured in 50mM tartrate buffer (pH 3, 25°C) by addition of 100$\mu$M $H_2O_2$ to a reaction mixture containing 0.5$\mu$M/ml MnP and 5-200$\mu$M $Mn^{2+}$. The formation of $Mn^{3+}$ is monitored spectrophotometrically at 238nm (Kmaitisuji et al., 2005).

To determine weather a particular enzyme system (as listed in Table 1) useful for producing a desired product contains unwanted activities from any other enzyme system (as listed in Table 1) producing a different unwanted product from the same or a different polymeric substrate, one can test for this activity using a specific polymeric substrate or artificial substrate as described above by using any of the methods as described above.

For example if it is suspected that a particular enzyme system containing cellulases as listed in Table 1 contains unwanted xylosidase activity (as listed in Table 1), the primary cellulase activity can be tested first using an artificial cellulose substrate and determine the amount of glucose liberated after addition of a defined amount of the enzyme system in a unit time. Once the cellulase activity of the preparation has been determined, the same enzyme preparation can be tested for xylosidase activity by contacting the enzyme system with an artificial xylan substrate and subsequently measuring the amount of xylose liberated by a defined amount of enzyme in unit time. By measuring the relative conversion rates for a defined time period with both the cellulase and xylosidase substrates using a defined enzyme amount, the specific activities for each substrate class can be calculated.

Useful substrates for determination of any enzymatic activity as listed in Table 1 are listed in Table 2.

In an alternative embodiment an approach to determine if an enzyme system contains any independent contaminating enzyme activity is to separate the components of said preparation by methods such as electrophoresis or chromatography. The individual components of the preparation can be detected using specific methods such as colorimetric staining or detection of constituents by absorbance or other methods known to those skilled in the art. The relative % distribution of proteinaceous constituents can be determined using quantitative methods such as densitometry or any other equivalent method known to those skilled in the art in conjunction with appropriate mathematical calculations.

*Determination of Sequence*

Prior to individual or serial enzymatic treatment steps required to liberate defined monomeric or oligomeric products (building blocks) from a raw or processed polymeric feedstock, soluble components of the raw polymeric feedstock are preferably separated by one or a number of pretreatment step(s).

In a preferred embodiment of the invention, the pretreatment step(s) to separate the soluble components of the raw or processed polymeric feedstock prior to enzymatic treatment is a combination of at least one physico-chemical pretreatment step and one or more washing step. According to another preferred embodiment of the invention, the washing step(s) to separate the soluble components from the raw or processed polymeric feedstock prior to enzymatic treatment is (are) preferentially performed with hydrophilic solvent(s), preferably aqueous solvent(s) such as water. As stated above, it has been found that the washing step(s) and physico-chemical pretreatment step(s) enhance the efficiency of the subsequent enzymatic treatment step(s).

According to a further preferred embodiment, a physico-chemical pretreatment step is only employed prior to the first enzymatic treatment step (step a) of the raw polymeric feedstock. Such physico-chemical pretreatment step may be combined with at least one washing step prior to the first enzymatic treatment step (step a) of the raw polymeric feedstock. Further preferred, pretreatment steps of the processed polymeric feedstock only comprise at least one washing step but no further physico-chemical pretreatment step.

Physico-chemical pretreatment steps for polymeric feedstocks may include without limitation hot water extraction, low temperature steam explosion, acid steam explosion, ammonia steam explosion, and sonication. They can be used to physically modify raw polymeric substrates in order to increase surface accessibility of plant fibers and decrease the crystallinity of the cellulose fraction (Puls *et al.,* 1985; Ramos *et al.,* 2005; Kinley *et al.,* 2005). Preferentially, the above mentioned pretreatment steps alter the physical properties of raw polymeric substrate structure in a way that renders the substrate more accessible to subsequent enzymatic steps but release either limited amounts or none of its chemical building blocks. In addition, they can be used to further remove soluble substances contained in the raw polymeric feedstock, before contacting the raw polymeric feedstock with an enzyme system to prevent contamination of the products of this enzymatic process step by soluble substances contained in the feedstock.

When two or more process steps are employed sequentially, the sequence of these process steps and thereby the sequence of adding enzyme mixtures as described in Table 1 depends on the specific composition of raw polymeric feedstock used. The sequence of process steps and enzyme systems is chosen in a way that minimizes dosage and costs of enzyme catalysts as well as the feedstock contact time leading to the release of the desired products. Additionally, chosen sequence steps should optimize purity as well as profitability of the monomeric or oligomeric reaction products released from the polymeric substrate. The sequence of process steps is therefore feedstock and product dependent.

The specific sequence of enzyme treatments necessary for the decomposition of a particular feedstock into its unit constituents can be determined empirically, even if the feedstock composition is unknown. Therefore, it is possible to digest the raw polymeric feedstock in separate treatment steps with a number of enzyme mixtures of different products as listed in Table 1. For each of the treatment steps the purity and composition of the resulting product stream is measured by using analytical methods known to the person skilled in the art comprising but not limited to spectroscopic and chromatographic methods as described previously for the analysis of the feedstock composition and for determining the enzymatic activity.

Using the results of these measurements, it is then possible to select the kinetically favorable enzyme mixture resulting in the purest product streams as the primary feedstock treatment step. After repeated washing of the insoluble remains resulting from this particular enzyme treatment, the remains are processed again with another number of enzyme mixtures, except with the enzyme mixture selected for the primary treatment. For all of these enzyme treatments the resulting product composition is determined by methods as described previously. The enzyme mixture resulting in the purest product stream is selected as the secondary treatment step of a particular feedstock. The remaining insoluble material derived from the second processing is again thoroughly washed and again tested with all remaining enzyme systems as described above. This process of analytically determining and selecting the purest product streams resulting from each of the remaining enzyme mixtures in Table 1 is repeated until the feedstock is either decomposed completely or the insoluble feedstock residues remaining after repeated enzyme treatments do not pose a major economical gain to the operator in comparison to the cost of further treatment options.

In another option the feedstock composition is determined by aforementioned analytical procedures prior to sequence selection for enzymatic treatment options. Thus, according to a preferred embodiment of the invention, the enzyme systems to be employed and their sequence of use are determined by first analyzing the raw polymeric feedstock.

One such preferred embodiment of the invention is directed to a method, in particular to determine the enzyme systems to be used and their sequence, wherein, preferably after separating soluble components from the raw polymeric substrate as described above, the insoluble raw polymeric feedstock is

(a) first treated in separate enzymatic treatments with each of a plurality of enzyme systems (mixtures) such as listed in Table 1;

(b) For each enzymatic treatment, the defined monomeric or oligomeric building blocks liberated from the raw polymeric feedstock are analyzed for purity of the defined monomeric or oligomeric building blocks, preferably after solid-liquid separation of the (soluble) defined monomeric or oligomeric building blocks from the (insoluble) raw polymeric feedstock;

(c) The enzyme system giving the highest purity is chosen for the first enzymatic treatment step according to step a) of claim 1;

(d) Optionally, the sequence of steps a) to c) is repeated with the remainder of the raw or processed polymeric feedstock in order to determine the enzyme system to be used in the subsequent enzymatic treatment step.

In an alternative embodiment, after separating soluble components from the raw or processed polymeric feedstock as described above, selective enzyme mixtures as listed in Table 1 are applied to target the feedstock constituent contributing the largest mass ratio to the feedstock composition. After enzymatic treatment, the purity of the resulting product stream has to be determined as previously described for the analytical determination of the feedstock components. It is desired, according to a preferred embodiment, that the purity is such that more than 75 wt.-%, preferably more than 90 wt.-%, more preferably more than 95 wt.-%, more preferably more than 99 wt.-% of the total solids content (preferably of the soluble fraction) consists of the defined monomeric or oligomeric building blocks.

If enzymatic decomposition of the largest mass feedstock component results in a pure product stream (as defined above), this treatment can be applied as the primary step for the feedstock processing. Washing of the resulting insoluble mash and subsequent solid-liquid separation of the particulate from the supernatant will prepare for the next processing steps. The remaining insolubles derived from the primary feedstock treatment will be treated with specific enzyme systems, such as the enzyme systems listed in Table 1, that target the feedstock constituents constituting the second largest mass ratio to the feedstock composition. The resulting product stream again has to be analyzed by said analytical methods to ascertain product purity before the selected enzymatic treatment can be deemed as the second treatment option for feedstock processing. The resulting insoluble residues of the secondary enzymatic treatment can then be washed and processed as described previously. Through iterative treatments with specific enzyme systems listed in Table 1, which always target the polymeric substrate constituting the largest mass ratio constituent in the remaining insoluble feedstock residue resulting from previous enzymatic treatment cycles, the feedstock can sequentially be decomposed into its unit constituents.

A further preferred aspect of the invention is directed to a method, in particular to determine the enzyme systems to be used and their sequence, wherein, preferably after separating soluble components from the raw polymeric feedstock as described above the insoluble raw polymeric feedstock is

(a) first treated in separate enzymatic treatments with each of a plurality of enzyme systems (mixtures) such as listed in Table 1 to determine the monomeric or oligomeric building blocks contributing the largest mass ratio in the raw polymeric feedstock;

(b) The defined monomeric or oligomeric building blocks contributing the largest mass ratio in the raw polymeric feedstock are analyzed for purity, preferably after separation from the raw polymeric feedstock;

(c) If the purity as determined is such that more than 75 wt.-%, preferably more than 90 wt.-%, more preferably more than 95 wt.%, more preferably more than 99 wt.-% of the total solids content consists of the defined monomeric or oligomeric building blocks, the respective enzyme system is chosen for the first enzymatic treatment step according to step a) of claim 1;

(d) If the purity determined according to step b) is lower than required according to step c), the defined monomeric or oligomeric building blocks contributing the next largest mass ratio in the raw polymeric feedstock are analyzed for purity, preferably after solid-liquid separation from the (insoluble) raw polymeric feedstock accordingly, until the purity satisfies the requirement according to step c) and the respective enzyme system is chosen for the first enzymatic treatment step according to step a) of claim 1;

(e) Optionally, the sequence of steps a) to d) is repeated with the remainder of the raw or processed polymeric feedstock in order to determine the enzyme system to be used in the subsequent enzymatic treatment step.

Yet a further preferred aspect of the invention is directed to a method, in particular to determine the enzyme systems to be used and their sequence, wherein the raw polymeric feedstock is

(a) first treated in separate enzymatic treatments with each of a plurality of enzyme or enzyme systems (mixtures) such as listed in Table 1 to determine the enzymatic treatment that leads to the highest yield of monomeric or oligomeric building blocks contained in the raw polymeric feedstock;

(b) Those enzymatic treatments are selected that yield a defined monomeric or oligomeric product with a purity of more than 75 wt.-%, preferably more than 90 wt.-%, more preferably more than 95 wt.-%, more preferably more than 99 wt.-% of the total solids (preferably after separation from the insoluble raw or processed polymeric feedstock);

(c) Among the remaining enzymatic treatments, the treatment with the highest yield of monomeric or oligomeric product is determined;

(d) Optionally the sequence of steps a) to c) is repeated with the remainder of the raw or processed polymeric feedstock in order to determine the enzyme system to be used in the subsequent enzymatic treatment step.

As stated above, according to a preferred aspect of the invention, the enzyme systems employed should be low in or essentially void of other or contaminating enzyme activities that liberate other than the intended monomeric or oligomeric building blocks from the raw or processed polymeric feedstock.

Thus according to a preferred embodiment, the enzyme system used in a particular enzymatic treatment step contains not more than 50%, preferably not more than 20%, more preferably not more than 10%, more preferably not more than 5%, more preferably not more than 2%, more preferably not more than 1% of contaminating (other) enzymatic activities, which have not been employed in a previous enzymatic treatment step using a different enzyme system or which can cause liberation of other defined monomeric or oligomeric building blocks that have not been liberated in previous enzymatic treatment steps, or, according to one further embodiment, which can only cause liberation of products from polymeric substrates that are initially essentially absent from the polymeric feedstock. The percentage of other or contaminating enzyme activities in the enzyme system may be calculated as set out above. "Essentially absent from", according to a preferred embodiment, means less than 20 wt.-%, preferably less than 10 wt.-%, preferably less than 5 wt.%, preferably less than 2 wt.-%, preferably less than 1 wt.-% of the total polymeric feedstock.

However, according to an advantageous and preferred embodiment of the invention, the enzyme system employed in a particular enzymatic treatment step contains as contaminating enzymatic activities one or more of such enzymatic activities, which have been employed in a previous enzymatic treatment step using a different enzyme system, or, according to a further embodiment, enzyme systems, which can only cause liberation of other monomeric or oligomeric building blocks from polymeric substrates that are initially essentially absent in the raw or processed polymeric feedstock. In other words, it has been found that when a particular monomeric or oligomeric building block has been previously liberated from the raw or processed polymeric feedstock in a previous enzymatic treatment step (or was not present in the raw polymeric feedstock from the beginning), it is not essential that the enzyme system used in a subsequent step is essentially free of the respective enzyme activity used as main activity in any of the previous enzymatic treatment step. One advantage of this embodiment is that less pure and thus less costly enzyme systems may be used in the second and following enzymatic treatment steps.

In a specific case of such a process the enzymatic conversion of xylan to xylose is performed in one process step after enzymatic processing of cellulose with subsequent removal of the product glucose in a previous process step. Hence, the enzyme mixtures in Table 1 designated for the processing of xylan to xylose may also contain any contaminating enzymatic activities, which are specific for the processing of cellulose.

Additionally, according to a further preferred embodiment of the invention, enzyme systems used for processing of a particular raw or processed polymeric feedstock constituent may contain contaminating (other) enzyme activities if these act on specific reaction intermediates resulting from the former enzymatic reactions and if the final end products are identical. In other words, a further preferred embodiment of the invention is directed to a method wherein the enzyme system used in a particular enzymatic treatment step has a main enzymatic activity (as described above) and contains at least one additional enzymatic activity which leads to the same defined monomeric or oligomeric building block(s) from the raw or processed polymeric feedstock as the main enzymatic activity of the enzyme system, in particular from a different polymeric substrate present in the raw or processed polymeric feedstock.

In a specific case the raw polymeric substrate contains both cellulose and starch (amylose) and the product of interest in the respective process step is glucose. Then the contamination of an enzyme mixture designated in Table 1 for the conversion of cellulose (cellulase activities) with accompanying side activities for the conversion of starch (amylase activities) as designated in Table 1 can be tolerated. The enzyme system therefore must not contain other enzyme activities above a certain ratio except for amylase activities. Here, the educts for the different enzymatic reactions may differ but the product in each case is glucose.

In the preferred case the said enzyme mixtures applied have to be essentially free of specific enzymatic activities that would result in the contamination of the resulting product stream.

In another specific case where glucose is a product of interest and the raw polymeric feedstock contains cellulose as a polymeric substrates (e.g. wheat straw), an enzyme mixture of cellulases is applied. Useful enzyme mixtures are designated in Table 1. Such cellulase mixtures can be used in conjunction with beta-glycosidases, glucohydrolases, and alpha- or beta-amylases as listed in Table 1 to convert the cellulose fraction of the raw polymeric feedstock to monomeric glucose units. In order to obtain a pure product stream of glucose, the enzyme mixture applied to the cellulose fraction has to be free of any hemicellulase activities, which encompass but are not limited to enzyme activities of arabinofuranosidase, arabinase, galactosidase, mannanase, mannanosidase, xylanase, and xylosidase. In this enzymatic process the polymeric cellulose will be transformed to soluble glucose units, which can be physically separated from the insoluble feedstock as described above. The remaining insoluble material can be processed further to produce various pentose sugars from the hemicellulose fraction.

In another specific case where arabinose and xylose are products of interest and the raw polymeric feedstock contains polymeric substrates comprising heterogeneous hemicellulose polymers such as arabinoxylan, an enzyme mixture designated in Table 1 for the conversion and mobilization of branched arabinose units is applied first. Subsequently, the soluble arabinose units are separated from the remaining insoluble feedstock before a second enzyme mixture designated in Table 1 is applied to mobilize the xylose units contained in xylan polymers. The soluble xylose units are then also separated from the remaining insoluble feedstock.

Enzymatic process steps can be combined with one or more pretreatment steps. Such pretreatment step(s) can be unselective to extract components of low commercial value that would otherwise contaminate high-value product streams from the process. Thus, according to one embodiment, one or more of the pretreatment steps are used to extract or otherwise remove defined components. Alternatively, selective one or more pretreatment steps can be used that provide a comparable selectivity to enzymatic process steps in solubilizing individual chemical components of the raw polymeric substrates. Thus, according to one embodiment, one or more pretreatment steps are used to increase the selectivity of the subsequent enzymatic treatment steps. Examples of such process steps would be solubilization of the lignin fraction of LCB by organic solvents such as ethanol or glycerol (Itoh *et al.,* 2003; Demirbas, A., 1998). These individual pretreatment steps and conditions are known to the person skilled in the art. In an additional alternative, said unselective pretreatment steps are applied to insoluble residues, which have been freed from contaminants by previous selective process steps. An example of such a process step is the complete hydrolysis of the protein fraction by acid treatment after selective solubilization of the hemicellulose, cellulose, and lignin fraction of LCB by aforesaid selective process steps.

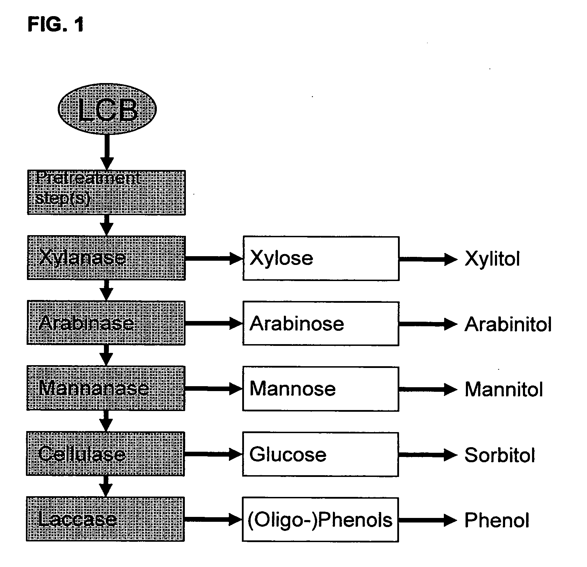

Another specific example for an embodiment of the invention is given in Figure 1. Thus, Figure 1 shows a process flow for the sequential enzymatic processing of LCB.

In this illustration (see Fig. 1) of processing of a substrate rich in cellulose, hemicellulose and lignins and with low amounts of proteins and lipids like wheat straw, corn stover, or softwood, solubilization of the various pentose components contained in the hemicellulose fraction is achieved by sequential treatment with xylanase, arabinase, and mannanase enzymes, which specifically liberate xylose, arabinose, and mannose sugars, respectively. Suitable enzymes and process conditions for providing optimal process conditions for the enzymes are known to the person skilled in the art. The soluble fraction is removed after every process step and the insolubles are contacted with the subsequent enzyme. After processing of the pentose fraction, a similar process step is added to convert cellulose into glucose using a mixture of exo- and endocellulases optionally in combination with cellobiase or beta-glucosidase to liberate glucose and cellobiose from the cellulosic fraction of the LCB substrate. These soluble reaction products are removed from the reaction mixture with the supernatant. Similarly, the lignin fraction remaining in the insoluble phase is converted into its various phenolic building blocks using specific enzyme systems, such as laccases and lignin peroxidases. These phenolic and oligophenolic compounds are then extracted from the reaction mixture with the supernatant or by solvent extraction. Process conditions to perform this process step are known to the person skilled in the art. Each of the individual products resulting from enzymatic conversion of hemicellulose, cellulose, lignin, or other LCB constituents could be isolated after each successive round of enzyme application (see Fig. 1). The resulting, essentially pure chemical building blocks of phenolics, pentose and hexose sugars could then be further processed to high-value commercial products (see Fig. 1). In general, according to one embodiment, the defined monomeric or oligomeric building blocks liberated from the raw or processed polymeric feedstock are purified and optionally further processed.

In another illustration, agricultural residues rich in proteins and lipids such as residues from the production of rape seed, sunflower or olive oils can be contacted subsequently with aforesaid hemicellulases and cellulases and optionally pectinases followed by contacting the residues of this process steps with an unspecific protease. Such unspecific proteases are known to the person skilled in the art and can be produced in large scale. The protease treatment solubilizes amino acid and peptides from the polymeric feedstock. These amino acids and peptides can be subsequently used as a mixture or separated into individual substances by methods known to the person skilled in the art.

In one case the products of interest are arabinose, xylose, glucose, oligophenylpropanoids, monolignols, and/or monophenolics and these products are produced by conversion of the raw polymeric feedstock wheat straw into its component constituents by a sequential enzymatic conversion.

In the primary step of such a sequential process, finely milled wheat straw (1kg wt., $0.2\mu$m) with an approximate moisture content of 5% w/w is placed in a steel container. A minimal amount of 2l water is added and mixed with the feedstock. The resulting slurry is left to soak for 4h at room temperature. Excess liquid is removed to leave approximately 200ml of the solution. The container is sealed and heated for 1 h to 121°C at 10bar pressure in a conventional sterilizing autoclave system (Puls *et al.,* 1985; Harms, 1989; Foody *et al.,* 1998). The vessel pressure is rapidly released and the content is allowed to cool to room temperature. Under these conditions the polymerization state of the feedstock components is reduced but only a minimal fraction of its components is released in soluble form. The resulting liquid phase (containing salts and minor amounts of various soluble components) and the insoluble phase (containing insoluble polymeric substrates such as cellulose, hemicellulose, and lignin) are separated by way of filtering, sieving, or centrifugation, and the insoluble phase is processed further.

In the next step, in order to mobilize arabinose components contained in the insoluble hemicellulose fraction, a mixture of alpha-L-arabinofuranosidases is applied. All of the reactions mentioned below are performed at a minimum of 40°C for 24h in 50mM phosphate buffer having a pH of 5-7. 0.08-1 g GH51 alpha-L-arabinofuranosidase from *Clostridium thermocellum* / kg of feedstock are added to hydrolyze the alpha-1,2/1,3-arabinofuranosyl moieties of arabinan and xylan (Taylor *et al.,* 2006). Subsequently, 0.08-1 g GH43 alpha-L-arabinofuranosidase from *Humicola insolens* / kg of feedstock are added to hydrolyze the alpha-1,5-arabinofuranosyl units (Sorensen *et a*/., 2006). Because of the specificity of the enzyme mixture, the resulting liquid phase contains mainly arabinose. The released and soluble arabinose units are separated from the insoluble marsh by filtering or centrifugation. The remaining insoluble fraction is retained for further processing.

In the next step, in order to convert insoluble xylan constituents into soluble xylose units, a mixture of xylanases and xylosidases is applied. All of the reactions are performed at a minimum of 40°C for 24h in 50mM phosphate buffer having a pH of 5-7. 0.08-1 g endo-1,4-beta-xylanase (GH10 or 11) from *Humicola insolens* /kg feedstock and 0.08-1g beta-xylosidase (GH3) from *Trichoderma reesei* / kg of feedstock are added to release xylo-oligosaccharides and xylanose units, respectively. Because of the specificity of the enzyme mixture, the resulting liquid phase contains mainly xylose. The soluble xylose is separated from the insoluble feedstock remains by filtering or centrifugation.

In the next step, in order to mobilize hexose sugars remaining in hemicellulose and cellulosic fractions, the respective insolubles are contacted with an optimized enzyme mixture containing endo- and exocellulases. All reactions are carried out at a minimum of 50°C for 16h in 50mM sodiumacetate buffer (pH 5-6). Mixtures of each 0.005-1g 1,4-beta-endoglucanases (Cel5A, Cel7B, Cel12A, Cel61A) and 1,4-beta-cellobiohydrolases (Cel7A, Cel6A) from *Trichoderma reesei* /kg insoluble feedstock are added to mobilize hexose sugars (Irwin *et al.,* 1993, Kim *et al.,* 1998). The efficacy and kinetics of cellulose conversion to monomeric sugar units is optionally enhanced by addition of 0.0005-0.01g cellobiose dehydrogenase from *Phanerocaete chrysosporium* in combination with 0.05-1g ferrocyanide and 0.0005-0.1g beta-glycosidase (10% wt enzyme mix) from *Aspergillus niger* / kg feedstock (Igarashi *et al.,* 1998, Rosgaard *et al.,* 2006). Because of the specificity of the enzyme mixture, the resulting liquid phase contains mainly hexoses, predominantly consisting of glucose. These are further separated by way of filtration or centrifugation from the fine particulate remains of the feedstock.

In the next step, insoluble lignin remaining after previous enzyme treatments is converted into its constituents by sequential contact with lignin peroxidase and laccase enzyme systems. Reactions with lignin peroxidase are carried out in a minimum of 50mM sodium tartrate (pH 3.5) and at a maximum temperature of 32°C. Highly polymeric lignin insolubles are oxidatively decomposed by contacting with each 0.5-1g of lignin peroxidase (LIP) from *Phanerocaete chrysosporium* / kg of feedstock (Ward *et al.,* 2003). To prevent catalytic inactivation of LIP (Compound III formation, Wariishi and Gold 1990) due to the presence of excess of $H_2O_2$ in the reaction mixture, a soluble enzymatic $H_2O_2$-generating system is used to provide a controlled and continuous environment for $H_2O_2$ formation. The $H_2O_2$-generating power of glyoxal oxidase (GLOX), a natural accessory enzyme working in synergy with lignin peroxidases (Kersten 1990) can be employed. The reaction of GLOX requires the same pH, ionic strength and temperature profile as described for LIP. The generation of $H_2O_2$ is induced by addition of 0.05-1g GLOX and 0.1-1g of the GLOX substrate methylglyoxal / kg of feedstock. In order to induce and enhance the oxidative decomposition of polymeric lignin by LIP, the redox mediator veratrylalcohol (3,4-dimethoxybenzyl alcohol) is added to proceed to completion (Ferapontova *et al.,* 2006). A more effective degradation of insoluble lignin can be achieved by adding 2g veratrylalcohol / kg of feedstock (Barr et *al.,* 1993). The main reaction products of the LIP-catalyzed oxidative decomposition of insoluble lignin are oligophenylpropanoids, while monolignols are only minor components of the product mixture.

In order to increase the amount of monophenolics in the product mixture, the LIP derived product mixture is further reacted with laccase (d'Acunzo *et al.,* 2002). The reaction is carried out at a minimum of 40°C for 6h in 100mM phosphate buffer having a pH of 5-6. 0.0004g laccase from *Trametes versicolor* and 0.0005g 2,2,6,6-tetramethylpiperidin-1-yloxy (TEMPO) are added as a redox mediator / kg feedstock (Arias *et al.,* 2003) to oxidize oligophenolics to monophenolic lignin units. Because of the specificity of the enzyme mixture, the resulting liquid phase contains mainly monophenolic lignin units. The resulting product mixture is separated from the remaining marsh by membrane filtration and simple centrifugation.

Literature

Hamsen, G. et *al.* (1989) Process for the treatment of biomass with steam, product thereby obtained and its use and reactor. EP 0187422A2

Chen, W.P., Matsuo, M., Yasui, T. (1986) Agric. Biol.Che,. 50, pp. 1183-1194

Arias, M.E., Arenas, M., Rodriguez, J., Solviveri, J., Ball, A.S., Hernandez, M. (2003) Kraft pulp biobleaching and mediated oxidation of a non-phenolic substrate by laccase from Streptomyces cyaneus CECT 3335. Appl.Envir.Microbiol. 69, pp. 1953-1958

D'Acunzo, F. Galli, C., Masci, B. (2002) Oxidation of phenols by laccase and laccase-mediator systems. Solubility and steric issues. Eur.J.biochem. 269, pp. 5330-5335

Barr, D., Sha, M.M., Aust, S.D. (1993) Veratrylalcohol-dependent production of molecular oxygen by Lignin peroxidase. J.Biol.Chem. 268, pp. 241-244

Currie, H.A., Perry, C.C. (2006) Resolution of complex monosaccharide mixtures from plant cell wall isolates by high pH anion exchange chromatography. J.Chromatography. 1128 (1-2), pp. 90-96

Demirbas, A. (1998) Aqueous glycerol delignification of wood chips and ground wood. Bioresource Technol. 63 (2), pp. 179-185

Irwin, D.C., Spezio, M., Walker, L.P., Wilson, D.B. (1993) Biotech.Bioengineer. 42, pp. 1002-1013.

Itoh, H., Wada, M., Honda, Y., Kuwahara, M., Watanabe, T. (2003) Bioorganosolve pretreatments for simultaneous saccharification and fermentation of beech wood by ethanolysis and white rot fungi. J.Biotechnol. 103, pp. 273-280

Igarashi, K., Samejima, M. Eriksson, K.-L. (1998) Cellobiose dehydrogenase enhances Phanerocaete chrysosporium cellobiohydrolase I activity by relieving product inhibition. Eur.J.Biochem. 253, pp. 101-106

Ferapontova, E.E., Castillo, J., Gorton, L. (2006) Bioelectrocatalytic properties of lignin peroxidase frpm Phanerocaete chrysosporium in reactions with phenols, catechols and lignin-model compounds. Biochem.Biophys.Acta 1760 (9), pp. 1343-54 Foody, B. *et al.* (1998) Pretreatment process for the conversion of cellulose to fuel ethanol. US 6,090,595

Kamm, B., Gruber, P.R., Kamm, M. (2006) Industrial processes and products Status quo and future direction. Biorefineries 1, pp. 1-39 Kamitsuiji, H., Watanabe, T., Honda, Y., Kuwahara, M. (2005) Direct oxidation of polymeric substrates by multifunctional manganese peroxidase isoenzyme from Pleurotus ostreatus without redox mediators. Biochem. J. 386, pp. 387-393.

Kaschemekat, J. Klose, M. (1985) Trennung der Komponenten eines Flüssigkeitsgemisches. DE 3410155C1

Kersten,P.J. (1990) Glyoxal oxidase of Phanerocaete chrysosporium: its characterization and activation by Ligninperoxidase Proc.Natl.Acad.Sci. 87, pp. 2936-2940

Kim, E., Irwin, D.C., Walker, L.O., Wilson, D.B. (1998) Factorial optimisation of a six-cellulase mixture. Biotech.Bioengineer. 58(5), pp. 494-501

Kinley, M.T, Krohn, B. Biomass conversion to alcohol using ultrasonic energy. US 200570136520A1

Lawford, H.G., Rousseau, J.D. (2003) Cellulosic fuel ethanol. Appl.Biochem and Biotechnol. 105, pp. 457-469

Lawford, H.G., Rousseau,J.D. (2003) Cellulosic fuel ethanol. Alternative fermentation process designs with wild-type and recombinant Zymomonas mobilis. Appl.Biochem.Biotechnol. 105, pp. 457-469

Lynd, L.R., van Zyl,W.H.v., McBride, J.E., Laser, M. (2005) Consolidated bioprocessing of cellulosic biomass: an update. Curr. Opin.Biotechnol. 16, pp. 577-583

Mammela, P. (2001) Phenolics in selected European hardwood species by liquid chromatography-electrospray ionization mass spectrometry. Analyst 126(9), pp. 1535-1538

Palla, G. (1981) C18 reversed-phase liquid chromatography determination of invert sugar, sucrose and raffinose. Anal.Chem. 53, pp. 1966-1967

Puls, J., Poutanen, K., Körner, H.-U., Viikari, L. (1985) Biotechnological utilization of wood carbohydrates after steaming pretreatment. Appl. Microbiol. Biotechnol. 22, pp. 416-423

Ramos, L.P., Silva, T.A., Martins, L.F., Satyanarayana, K.G. (2005) Conversion of lignocellulosics to fules, chemicals and environmentally-friendly materials. Metals and Processes 117, pp. 299-318

Rosgaard, L., Peterson, S., Cherry, J.R., Harris, P., Meyer, A.S. (2006) Efficiency of new fungal cellulose systems in boosting enzymatic degradation of barley straw lignocellulose. Biotechnol.Prog. 22(2), pp. 493-498

Saha, B.C., Enzymes as biocatalysts for conversion of lignocellulosic biomass to fermentable sugars (2005) in Handbook of industrial biocatalysis, ed. Ching T. Hou, CRC Press, Chapter 24, pp. 1-12

Smirnov, S.A., Korovela, O.V., Gavrilova, V.P., Belova, A.B., Klyachko, N.L. (2001) Laccases for basidomyces: Physicochemical characteristics and substrate specificity towards methoxyphenolic compounds. Biochem.(Moscow) 66(7), pp. 774-779

Sorensen, H., Pederson, S., Viksoe-Nielsen, A. *et al.* (2006) Hydrolysis of arabinoxylan. WO 2006114095A1

Taylor, E. Smith, N., Turkenburg, J. et al..(2006) Structural insights into the ligand specificity of a thermostable family 51 arabinofuranosidase, Araf51, from Clostridium thermocellum. Biochem.J. 395, pp. 31-37

Ward, G., Hadar, Y., Bilkis, I., Dosoretz, C. (2003) Mechanistic features of lignin peroxidase-catalysed oxidation of substituted phenols and 1,2-dimethoxyarenes. J.Biol.Chem. 278, pp. 39726-39734

Wariishi, H., Gold, M.H. (1990) Lignin Peroxidase Compound III. Mechanism of formation and decomposition. J.Biol.Chem. 265, pp. 2070-2077

Wood, T.M. and Baht, K.M., Methods for measuring cellulose activities. Methods in Enzymology. 160, pp. 87-112

Table 1 Enzyme systems

| Product | Polymeric Substrate | Enzyme activity | Enzyme mixture numbers |
|---|---|---|---|
| **1-Acylglycerophosphocholine** | Phosphatidylcholine | Phospholipase | 1 |
| **1,5-Anhydro-D-fructose+ D-glucose** | Alpha-glucan | Exo-alpha-1,4-D-glucan lyase | 1 |
| **Alcohol + acetate** | Xyloglucan | Acetylesterase | 1 |
| | Rhamnogalacturonan | | |
| **Amino acids** | Proteins | Protease | 1 |
| **Arabinose** | Arabinan | Arabinofuranosidase | 2,5 |
| | Arabinoxylan | Endo-alpha-1,3-L-arabinanase | 1,3,4 |
| | Xyloglucan | Endo-alpha-1,5-L-arabinanase | 1 |
| | | Exo-alpha-1,3-L-arabinanase | 1,2,3 |
| | | Exo-alpha-1,5-L-arabinanase | 1,2,5 |
| **Choline** | Acetic esters Choline esters | Acetylcholinesterase | 1,2 |
| | | Cholinesterase | 1,3 |
| **D-Xylonate** | Xylono-1,4-lactone | Xylono-1,4-lactonase | 1 |
| **Diacylglycerol** | Triglyceridester | Triacylglycerollipase | 1 |
| **Fucose** | Xyloglucan | Endo-alpha-1,2-L-fucosidase | 1,2 |
| | | Exo-alpha-1,2-L-fucosidase | 1,2,3,4 |
| | Pectin | Pectinase | 1,3 |
| **Galactose** | Galactan | Endo-beta-1,4-D-galactosidase | 1 |
| | Galactomannan | Exo-beta-1,4-D-galactosidase | 1,2 |
| | Xyloglucan | | |
| **Gallate** | Digallate | Acylglycerollipase | 1,3 |
| | Glycerol monoesters of long-chain fatty acids | Tannase | 1,2 |

(continued)

| Product | Polymeric Substrate | Enzyme activity | Enzyme mixture numbers |
|---|---|---|---|
| **Glucose** | Cellulose | Cellulase | 1 ,2,3,4,5,6,7,8 |
| | Glucomannan | Alpha-amylase | 1,2,3,4,6 |
| | Glucoronoxylan | Beta-amylase | 1,2,3,5,6 |
| | Xyloglucan | Beta-glucosidase | 1,2,3,4,6 |
| | | Cellobiohydrolase I | 1,3,4,6,7 |
| | | Cellobiohydrolase II | 2,3,4,6,8 |
| | | Endo-beta-1-4-D-glucanase | 1,2,3,4,5,6 |
| | | Endoglucanase I | 1,2,4,6 |
| | | Endoglucanase II | 1,2,4,5,6 |
| | | Endoglucanase III | 1,2,3,5,6 |
| | | Endoglucanase IV | 1,2,3,5,6 |
| | | Endoglucanase V | 1,3,4,6 |
| | | Endoglucanase VI | 1,3,4,6 |
| | | Endoglucanase VII | 1,3,4,6 |
| | | Exo-beta-1,4-D-glucanase | 1,2,3,4,5,6,7,8 |
| | | Glucohydrolase | 1,2,3,4,6 |
| **Glucose** | Starch | Alpha-amylases | 1,2 |
| | | Beta-amylases | 1,3 |
| **Glycerophosphocholine** | 2-Lysophosphatidylcholine | Lysophospholipase | 1 |
| **L-Arabinonate** | L-Arabinono-1,4-lactone | L-Arabinonolactonase | 1 |
| **Long-chain alcohol** | Wax ester | Wax-ester hydrolase | 1 |
| **Long-chain-fatty acid** | Long-chain-fatty-acyl ethyl ester | Fatty-acyl-ethyl-ester synthase | 1 |
| **Mannose** | Galactomannan Mannan | Beta-1,4-D-mannosidase | 2 |
| | | Endo-beta-1,4-D-mannanase | 1 |
| | | Exo-beta-1,4-D-mannanase | 1,2,3 |
| **Methanol + pectate** | Pectin | Pectinesterase | 1,2,3 |
| | | Pectin demethoxylase | 1 |
| | | Pectin methoxylase | 1,2 |

(continued)

| Product | Polymeric Substrate | Enzyme activity | Enzyme mixture numbers |
|---|---|---|---|
| **Oligolignan, monolignole, phenolic compounds, oligophenylpropanoids** | Lignin | Laccase (TEMPO) | 1,2,6 |
| | | Lignin peroxidase (Veratrylalcohol) + Glyoxal oxidase (primary aldehydes or methylglyoxal) | 1,3,4 |
| | | Manganese peroxidase (Mn $^{2+}$ organic acids) | 1,2,3,5 |
| **Oligopeptides** | Proteins | Amino-peptidase | 1,2,8 |
| | | Carboxy-peptidase | 1,3,8 |
| | | Carboxyl-proteinase | 1,4 |
| | | Endo-peptidase | 1,4,5,6,7 |
| | | Exo-peptidase | 1,2,3,8 |
| | | Metallo-proteinase | 1,5 |
| | | Serin-proteinase | 1,6 |
| | | Thiol-proteinase | 1,7 |
| **Oligosaccharides with terminal 4-deoxy-alpha-D-galact-4-enuronosyl groups** | Alpha-1,4-D-galacturonan | Pectate lyase (alpha-1,4-D-endopolygalacturonic acid lyase) | 1 |
| **Phytol** | Chlorophyll | Chlorophyllase | 1 |
| **Ribonucleotides** | RNA | Endoribonuclease | 1,2,3 |
| | | Exoribonuclease | 1,2,4,5 |
| | | Ribonuclease | 1,3,4,5 |
| **Sterol** | Steryl ester | Sterol esterase | 1,2 |
| | | Triterpenol esterase | 1,3 |
| **Xylose** | Arabinoxylan Glucoronoxylan Xylan Xyloglucan | Endo-beta-1,3-D-xylanase | 1,3,6 |
| | | Endo-alpha-1,6-D-xylosidase | 1,2 |
| | | Endo-beta-1,4-D-xylanase | 1,2,3 |
| | | Exo-alpha-1,6-D-xylanase | 1,2,7 |
| | | Exo-beta-1,3-D-xylanase | 1,3,5,6 |
| | | Exo-beta-1,4-D-xylanase | 1,2,3,4 |
| | | Xylosidase | 1,2,3 |
| **Exemplary enzyme combinations for the generation of a specific product obtained from a specific polymeric feedstock constituent are denoted Individually by numbers ranging from 1 to 8.** | | | |

Table 2

| Substrate | Sigma Cat. Number | Enzyme | Prod |
|---|---|---|---|
| Cellulose | C6288 | Endo-cellulase, Exo-cellulase, β-Glycosidase | Glucose |
| Cellodextrins | C4642 | Endo-cellulase, Exo-cellulase, β-Glycosidase | Glucose |
| β-Methlylumbelliferyl-oligosacharides | M6018 M60 18 | Endo-cellulase, Exo-cellulase, β-Glycosidase | Glucose |
| p-Nitrophenol-oligosaccharides | N0145 | Endo-cellulase, Exo-cellulase β-Glycosidase | Glucose |
| CMC | C9481 | Endocellulase | Oligoscaccharides |
| Avicel PH-101 | 11365 | Exocellulase | Glucose |
| 4-Nitrophenyl-ß-D-cellobioside | N5759 | | Glucose and pNP |
| Xylan | X4252 | Xylanase, xylanosidase | Xylose |
| 4-Nitrophenyl-ß-D-xylopyranoside | N2132 | Cellulase | Xylose and pNP |
| Mannan from yeast | M7504 | Mannanase | Mannanoside and mannose |
| D-Galacto-D-mannan from *Ceratonia siliqua Ceratonia silique* | 48230 | Galactase and mannanase | Galactose and mannose |
| 4-Nitrophenyl $\alpha$-L-arabinofuranoside | N3641 | Arabinofucosidase | Arabinose and pNP |
| 2,3-Dimethoxybenzyl alcohol (veratrylalcohol) | 38700 | Lignin peroxidase (LIP) | Veratrylalcohol radical cation |
| Lignin, hydrolytic | 371076 | Lignin peroxidase (LIP) | Phenylpropanoids |
| Lignin, organosolv | 371017 | Lignin peroxidase (LIP) | Phenylpropanoids |
| 2,2'-Azino-bis(3-ethylbenzothiazoline-6-sulfonic acid (ABTS) | A1227 | Lignin peroxidase (LIP) | ABTS radical cation |
| 2,2'-Azino-bis(3-ethylbenzothiazoline-6-sulfonic acid (ABTS) | A1227 | Laccase | ABTS radical cation |
| Manganesechloride (MnCl$_2$) | 416479 | Manganeseperoxidase (MnP) | Mn$^{3+}$ ion |

**Claims**

1. Method for the enzymatic treatment of raw polymeric feedstock comprising the following steps:

   a. treating the insoluble raw polymeric feedstock with an enzyme system in order to liberate defined soluble monomeric or oligomeric building blocks from the raw polymeric feedstock;
   b. separating the defined soluble monomeric or oligomeric building blocks produced in step a) from the remainder of the insoluble raw polymeric feedstock.

2. The method of claim 1, wherein prior to step a) soluble components are separated from the raw polymeric feedstock.

3. The method of claim 2, wherein the separation of soluble components from the raw polymeric feedstock is performed using one or more washing step(s) and/or one or more physico-chemical treatment step(s).

4. The method of claim 1, wherein the enzyme system used in step a) contains not more than 50%, preferably not more than 20%, more preferably not more than 10%, more preferably not more than 5%, more preferably not more than 2%, more preferably not more than 1 % of other enzyme activities apart from the enzyme activity resulting in liberation of said defined soluble monomeric or oligomeric building blocks from the insoluble raw polymeric feedstock according to step a).

5. The method according to any one of the preceding claims, wherein steps a) and b) are repeated one or more times with different enzyme systems in order to liberate from the remainder of the raw polymeric feedstock (processed polymeric feedstock) other defined soluble monomeric or oligomeric building blocks.

6. The method according to claim 5, wherein prior to repetition of step a) the separation of soluble components from the processed polymeric feedstock is performed using one or more pretreatment step, preferably one or more washing step.

7. The method according to any one of the preceding claims, wherein the enzymatic treatment is performed in an aqueous medium, said defined monomeric or oligomeric building blocks liberated from the raw or processed polymeric feedstock are soluble in the aqueous medium and the separation according to step b) is performed by liquid/solid separation of the soluble building blocks in the aqueous medium from the remainder of the insoluble raw or processed polymeric feedstock.

8. The method according to any one of the preceding claims, wherein the defined monomeric or oligomeric building blocks according to step a) are chosen from one of the group consisting of glucose, xylose, arabinose, galactose, mannose, amino acids or phenolic compounds or other products listed in Table 1.

9. The method according to any one of the preceding claims, wherein the enzyme system used in a particular enzymatic treatment step contains not more than 50%, preferably not more than 20%, more preferably not more than 10%, more preferably not more than 5%, more preferably not more than 2%, more preferably not more than 1 % of contaminating enzymatic activities, which have not been employed in a previous enzymatic treatment step using a different enzyme system or which can cause liberation of other defined monomeric or oligomeric building blocks, which have not been liberated in previous enzymatic treatment steps, or which can only cause liberation of products from polymeric substrates that are initially essentially absent from the polymeric feedstock.

10. The method according to any one of the preceding claims, wherein the raw polymeric feedstock comprises cellulose and hemicellulose as polymeric substrates and the enzyme system used in a particular enzymatic treatment step has cellulase activity, and optionally beta-glycosidase, glucohydrolase and/or alpha- or beta-amylase activity, but is essentially free of hemicellulase activity.

11. The method according to any one of the preceding claims, wherein the enzyme system used in a particular enzymatic treatment step contains as contaminating enzymatic activities one or more of such enzymatic activities, which have been employed in a previous enzymatic treatment step using a different enzyme system or which can only cause liberation of other monomeric or oligomeric building blocks from polymeric substrates that are initially essentially absent in the raw polymeric feedstock.

12. The method according to any one of the preceding claims, wherein the enzyme system used in a particular enzymatic treatment step has a first enzymatic activity and contains at least one additional enzymatic activity, which leads to the same defined monomeric or oligomeric building block(s) from the raw or processed polymeric feedstock as the first enzymatic activity of the enzyme system, in particular from a different polymeric substrate present in the raw polymeric feedstock.

13. The method according to any one of the preceding claims, wherein the insoluble raw or processed polymeric feedstock is subjected to a selective or unselective physico-chemical treatment step prior to step a) or prior to repetition of step a) according to claim 6.

14. The method according to claim 10, wherein the physico-chemical treatment step comprises a treatment with aqueous solvents, organic solvents, or any combination or mixtures of these preferably with ethanol or glycerol.

15. The method according to any of the preceding claims, wherein the enzyme systems to be employed and their sequence of use is determined by first analyzing the raw polymeric feedstock.

16. The method according to claim 12, wherein, in order to determine the enzyme systems to be used and their sequence, the insoluble raw polymeric feedstock is

a. first treated in separate enzymatic treatments with each of a plurality of enzyme systems (mixtures) such as listed in Table 1;

b. for each enzymatic treatment the defined monomeric or oligomeric building blocks liberated from the raw polymeric feedstock are analyzed for purity of the defined monomeric or oligomeric building blocks after separation of the defined monomeric or oligomeric building blocks from the raw polymeric feedstock;

c. the enzyme system giving the highest purity is chosen for the first enzymatic treatment step according to step a) of claim 1;

d. optionally the sequence of steps a) to c) is repeated with the remainder of the raw or processed polymeric feedstock in order to determine the enzyme system to be used in the subsequent enzymatic treatment step.

17. The method according to claim 12, wherein, in order to determine the enzyme systems to be used and their sequence, the insoluble raw polymeric feedstock is

a. first treated in separate enzymatic treatments with each of a plurality of enzyme systems (mixtures) such as listed in Table 1 to determine the monomeric or oligomeric building blocks contributing the largest mass ratio in the raw polymeric feedstock;

b. the defined monomeric or oligomeric building blocks contributing the largest mass ratio in the raw polymeric feedstock are analyzed for purity after separation from the raw polymeric feedstock;

c. If the purity as determined is such that more than 75 wt.-%, preferably more than 90 wt.-%, more preferably more than 95 wt.-%, more preferably more than 99 wt.-% of the total solids content consists of the defined monomeric or oligomeric building blocks, the respective enzyme system is chosen for the first enzymatic treatment step according to step a) of claim 1;

d. If the purity determined according to step b) is lower than required according to step c), the defined monomeric or oligomeric building blocks contributing the next largest mass ratio in the raw polymeric feedstock are analyzed for purity after separation from the raw polymeric feedstock accordingly, until a purity satisfies the requirement according to step c) and the respective enzyme system is chosen for the first enzymatic treatment step according to step a) of claim 1;

e. optionally the sequence of steps a) to d) is repeated with the remainder of the raw or processed polymeric feedstock in order to determine the enzyme system to be used in the subsequent enzymatic treatment step.

18. The method according to claim 12, wherein, in order to determine the enzyme systems to be used and their sequence, the raw polymeric feedstock is

a. first treated in separate enzymatic treatments with each of a plurality of enzyme systems (mixtures) such as listed in Table 1 to determine the enzymatic treatment that leads to the highest yield of monomeric or oligomeric building blocks contained in the raw polymeric feedstock;

b. select those enzymatic treatments that yield a defined monomeric or oligomeric product wit a purity of more than 75 wt.-%, preferably more than 90 wt.-%, more preferably more than 95 wt.%, more preferably more than 99 wt.-% of the total solids;

c. determine among the remaining enzymatic treatments the treatment with the highest yield of monomeric or oligomeric product;

d. optionally the sequence of steps a) to c) is repeated with the remainder of the raw or processed polymeric feedstock in order to determine the enzyme system to be used in the subsequent enzymatic treatment step.

**FIG. 1**

**European Patent Office**

## EUROPEAN SEARCH REPORT

Application Number

EP 07 01 1507

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 884 391 A (IOGEN CORP [CA]) 16 December 1998 (1998-12-16) * page 6; example 3 * | 1-4,7,8, 10,14,15 | INV. C12P19/02 C12S3/04 C13K1/02 |
| X | US 6 586 212 B1 (BUCHANAN CHARLES M [US] ET AL) 1 July 2003 (2003-07-01) * abstract * * column 4 * | 1-10,14, 15 | C13K1/06 C07H1/08 |
| X | US 4 089 745 A (ANTRIM RICHARD LEE ET AL) 16 May 1978 (1978-05-16) * abstract * | 1-4,7,8, 10,14,15 | ADD. C12P19/12 C12P19/14 C12P19/16 |
| X | WO 03/093420 A (ATHENIX CORP [US]; DUCK NICHOLAS B [US]; CARR BRIAN [US]; KOZIEL MICHA) 13 November 2003 (2003-11-13) * examples 1,2 * | 1-4,7,8, 10,14,15 | C12P19/18 C12P19/20 C12P19/22 C12P19/24 |
| X | EP 0 406 617 A (INT PAPER CO [US]) 9 January 1991 (1991-01-09) * abstract * | 1-10,14, 15 | |
| X | WO 99/11672 A (DU PONT [US]; FITCHETT COLIN STANLEY [GB]) 11 March 1999 (1999-03-11) * the whole document * | 1-4,7,8, 10,14,15 | TECHNICAL FIELDS SEARCHED (IPC) C12P C13K |
| X | US 2002/195213 A1 (IZUMI YOSHIYA [JP] ET AL) 26 December 2002 (2002-12-26) * the whole document * | 1-4,7,8, 10,14,15 | |
| X | EP 0 062 027 A (STEYRERMUEHL PAPIER [AT]) 6 October 1982 (1982-10-06) * the whole document * | 1-4,7,8, 10,14,15 | |

-/--

The present search report has been drawn up for all claims

| Place of search | Date of completion of the search | Examiner |
|---|---|---|
| The Hague | 4 January 2008 | van Voorst, Frank |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

**EUROPEAN SEARCH REPORT**

Application Number

EP 07 01 1507

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YOON ET AL: "Enzymatic production of pentoses from the hemicellulose fraction of corn residues" LEBENSMITTEL WISSENSCHAFT UND TECHNOLOGIE, ACADEMIC PRESS, LONDON, GB, vol. 39, no. 4, May 2006 (2006-05), pages 388-392, XP005234767 ISSN: 0023-6438 * the whole document * ----- | 1-4,7,8, 10,14,15 | |
| X | GASPAR ET AL: "Corn fiber as a raw material for hemicellulose and ethanol production" PROCESS BIOCHEMISTRY, ELSEVIER, NL, vol. 42, no. 7, July 2007 (2007-07), pages 1135-1139, XP022138704 ISSN: 1359-5113 * the whole document * ----- | 1-4,7,8, 10,14,15 | |
| X | SORENSEN ET AL: "Synergistic enzyme mechanisms and effects of sequential enzyme additions on degradation of water insoluble wheat arabinoxylan" ENZYME AND MICROBIAL TECHNOLOGY, STONEHAM, MA, US, vol. 40, no. 4, 16 February 2007 (2007-02-16), pages 908-918, XP005892515 ISSN: 0141-0229 * the whole document * ----- | 1-4,7,8, 10,14,15 | TECHNICAL FIELDS SEARCHED (IPC) |

The present search report has been drawn up for all claims

| Place of search | Date of completion of the search | Examiner |
|---|---|---|
| The Hague | 4 January 2008 | van Voorst, Frank |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                     EP 07 01 1507

This annex lists the patent family members relating to the patent documents cited in the above-mentioned European search report.
The members are as contained in the European Patent Office EDP file on
The European Patent Office is in no way liable for these particulars which are merely given for the purpose of information.

04-01-2008

| Patent document cited in search report | | Publication date | Patent family member(s) | | Publication date |
|---|---|---|---|---|---|
| EP 0884391 | A | 16-12-1998 | CA | 2240035 A1 | 09-12-1998 |
| | | | CN | 1201832 A | 16-12-1998 |
| | | | DE | 69803145 D1 | 21-02-2002 |
| | | | DE | 69803145 T2 | 21-11-2002 |
| | | | US | 6090595 A | 18-07-2000 |
| | | | US | 5916780 A | 29-06-1999 |
| US 6586212 | B1 | 01-07-2003 | US | 2003207407 A1 | 06-11-2003 |
| US 4089745 | A | 16-05-1978 | NONE | | |
| WO 03093420 | A | 13-11-2003 | AU | 2003228765 A1 | 17-11-2003 |
| | | | CA | 2484118 A1 | 13-11-2003 |
| | | | EP | 1511848 A2 | 09-03-2005 |
| | | | JP | 2005523720 T | 11-08-2005 |
| | | | US | 2004005674 A1 | 08-01-2004 |
| | | | US | 2007218530 A1 | 20-09-2007 |
| EP 0406617 | A | 09-01-1991 | AU | 621231 B2 | 05-03-1992 |
| | | | AU | 5762390 A | 03-01-1991 |
| | | | BR | 9002953 A | 20-08-1991 |
| | | | CA | 2019411 A1 | 22-12-1990 |
| | | | JP | 3040887 A | 21-02-1991 |
| | | | NO | 902756 A | 27-12-1990 |
| | | | NZ | 234156 A | 26-03-1992 |
| | | | PT | 94450 A | 08-02-1991 |
| | | | ZA | 9004441 A | 27-03-1991 |
| WO 9911672 | A | 11-03-1999 | AU | 9122398 A | 22-03-1999 |
| | | | CN | 1268955 A | 04-10-2000 |
| | | | EP | 1015498 A1 | 05-07-2000 |
| | | | ZA | 9807913 A | 29-02-2000 |
| US 2002195213 | A1 | 26-12-2002 | US | 2002174962 A1 | 28-11-2002 |
| EP 0062027 | A | 06-10-1982 | AT | 371499 B | 27-06-1983 |
| | | | AT | 139481 A | 15-11-1982 |
| | | | DD | 202180 A5 | 31-08-1983 |
| | | | DE | 3266282 D1 | 24-10-1985 |
| | | | FI | 820957 A | 26-09-1982 |

EPO FORM P0459

For more details about this annex : see Official Journal of the European Patent Office, No. 12/82

## REFERENCES CITED IN THE DESCRIPTION

### Patent documents cited in the description

- EP 0187422 A2
- US 6090595 A
- DE 3410155 C1
- US 200570136520 A1
- WO 2006114095 A1

### Non-patent literature cited in the description

- **CHEN, W.P. ; MATSUO, M. ; YASUI, T.** *Agric. Biol.Che,* 1986, vol. 50, 1183-1194
- **ARIAS, M.E. ; ARENAS, M. ; RODRIGUEZ, J. ; SOLVIVERI, J. ; BALL, A.S. ; HERNANDEZ, M.** Kraft pulp biobleaching and mediated oxidation of a non-phenolic substrate by laccase from Streptomyces cyaneus CECT 3335. *Appl.Envir.Microbiol.,* 2003, vol. 69, 1953-1958
- **D'ACUNZO, F. ; GALLI, C. ; MASCI, B.** Oxidation of phenols by laccase and laccase-mediator systems. Solubility and steric issues. *Eur.J.biochem.,* 2002, vol. 269, 5330-5335
- **BARR, D. ; SHA, M.M. ; AUST, S.D.** Veratrylalcohol-dependent production of molecular oxygen by Lignin peroxidase. *J.Biol.Chem.,* 1993, vol. 268, 241-244
- **CURRIE, H.A. ; PERRY, C.C.** Resolution of complex monosaccharide mixtures from plant cell wall isolates by high pH anion exchange chromatography. *J.Chromatography,* 2006, vol. 1128 (1-2), 90-96
- **DEMIRBAS, A.** Aqueous glycerol delignification of wood chips and ground wood. *Bioresource Technol.,* 1998, vol. 63 (2), 179-185
- **IRWIN, D.C. ; SPEZIO, M. ; WALKER, L.P. ; WILSON, D.B.** *Biotech.Bioengineer.,* 1993, vol. 42, 1002-1013
- **ITOH, H. ; WADA, M. ; HONDA, Y. ; KUWAHARA, M. ; WATANABE, T.** Bioorganosolve pretreatments for simultaneous saccharification and fermentation of beech wood by ethanolysis and white rot fungi. *J.Biotechnol.,* 2003, vol. 103, 273-280
- **IGARASHI, K. ; SAMEJIMA, M ; ERIKSSON, K.-L.** Cellobiose dehydrogenase enhances Phanerocaete chrysosporium cellobiohydrolase I activity by relieving product inhibition. *Eur.J.Biochem.,* 1998, vol. 253, 101-106
- **FERAPONTOVA, E.E. ; CASTILLO, J. ; GORTON, L.** Bioelectrocatalytic properties of lignin peroxidase frpm Phanerocaete chrysosporium in reactions with phenols, catechols and lignin-model compounds. *Biochem.Biophys.Acta,* 2006, vol. 1760 (9), 1343-54
- **KAMM, B. ; GRUBER, P.R. ; KAMM, M.** Industrial processes and products Status quo and future direction. *Biorefineries,* 2006, vol. 1, 1-39
- **KAMITSUIJI, H. ; WATANABE, T. ; HONDA, Y. ; KUWAHARA, M.** Direct oxidation of polymeric substrates by multifunctional manganese peroxidase isoenzyme from Pleurotus ostreatus without redox mediators. *Biochem. J.,* 2005, vol. 386, 387-393
- **KERSTEN,P.J.** Glyoxal oxidase of Phanerocaete chrysosporium: its characterization and activation by Ligninperoxidase. *Proc.Natl.Acad.Sci.,* 1990, vol. 87, 2936-2940
- **KIM, E. ; IRWIN, D.C. ; WALKER, L.O. ; WILSON, D.B.** Factorial optimisation of a six-cellulase mixture. *Biotech.Bioengineer.,* 1998, vol. 58 (5), 494-501
- **LAWFORD, H.G. ; ROUSSEAU, J.D.** Cellulosic fuel ethanol. *Appl.Biochem and Biotechnol.,* 2003, vol. 105, 457-469
- **LAWFORD, H.G. ; ROUSSEAU,J.D.** Cellulosic fuel ethanol. Alternative fermentation process designs with wild-type and recombinant Zymomonas mobilis. *Appl.Biochem.Biotechnol.,* 2003, vol. 105, 457-469
- **LYND, L.R. ; VAN ZYL,W.H.V. ; MCBRIDE, J.E. ; LASER, M.** Consolidated bioprocessing of cellulosic biomass: an update. *Curr. Opin.Biotechnol.,* 2005, vol. 16, 577-583
- **MAMMELA, P.** Phenolics in selected European hardwood species by liquid chromatography-electrospray ionization mass spectrometry. *Analyst,* 2001, vol. 126 (9), 1535-1538
- **PALLA, G.** C18 reversed-phase liquid chromatography determination of invert sugar, sucrose and raffinose. *Anal.Chem.,* 1981, vol. 53, 1966-1967
- **PULS, J. ; POUTANEN, K. ; KÖRNER, H.-U. ; VIIKARI, L.** Biotechnological utilization of wood carbohydrates after steaming pretreatment. *Appl. Microbiol. Biotechnol.,* 1985, vol. 22, 416-423

- **RAMOS, L.P. ; SILVA, T.A. ; MARTINS, L.F. ; SATYANARAYANA, K.G.** Conversion of lignocellulosics to fules, chemicals and environmentally-friendly materials. *Metals and Processes,* 2005, vol. 117, 299-318
- **ROSGAARD, L. ; PETERSON, S. ; CHERRY, J.R. ; HARRIS, P. ; MEYER, A.S.** Efficiency of new fungal cellulose systems in boosting enzymatic degradation of barley straw lignocellulose. *Biotechnol.Prog.,* 2006, vol. 22 (2), 493-498
- Enzymes as biocatalysts for conversion of lignocellulosic biomass to fermentable sugars. **SAHA, B.C.** Handbook of industrial biocatalysis. CRC Press, 2005, vol. 24, 1-12
- **SMIRNOV, S.A. ; KOROVELA, O.V. ; GAVRILO-VA, V.P. ; BELOVA, A.B. ; KLYACHKO, N.L.** Laccases for basidomyces: Physicochemical characteristics and substrate specificity towards methoxyphenolic compounds. *Biochem.(Moscow,* 2001, vol. 66 (7), 774-779
- **TAYLOR, E. ; SMITH, N. ; TURKENBURG, J. et al.** Structural insights into the ligand specificity of a thermostable family 51 arabinofuranosidase, Araf51, from Clostridium thermocellum. *Biochem.J.,* 2006, vol. 395, 31-37
- **WARD, G. ; HADAR, Y. ; BILKIS, I. ; DOSORETZ, C.** Mechanistic features of lignin peroxidase-catalysed oxidation of substituted phenols and 1,2-dimethoxyarenes. *J.Biol.Chem.,* 2003, vol. 278, 39726-39734
- **WARIISHI, H. ; GOLD, M.H.** Lignin Peroxidase Compound III. Mechanism of formation and decomposition. *J.Biol.Chem.,* 1990, vol. 265, 2070-2077
- **WOOD, T.M. ; BAHT, K.M.** Methods for measuring cellulose activities. *Methods in Enzymology,* vol. 160, 87-112